(19) Europäisches Patentamt
European Patent Office
Office européen des brevets

(11) **EP 0 747 385 B1**

(12) **EUROPEAN PATENT SPECIFICATION**

(45) Date of publication and mention of the grant of the patent:
**14.08.2002 Bulletin 2002/33**

(51) Int Cl.[7]: **C07F 9/655**, A61K 31/66, C07F 9/6558

(21) Application number: **96109044.6**

(22) Date of filing: **05.06.1996**

(54) **Prodrugs of paclitaxel derivatives**

Prodrugs von Paclitaxel-Derivaten

Promédicaments de dérivés du paclitaxel

(84) Designated Contracting States:
**AT BE CH DE DK ES FI FR GB GR IE IT LI LU MC NL PT SE**

(30) Priority: **06.06.1995 US 469247**

(43) Date of publication of application:
**11.12.1996 Bulletin 1996/50**

(73) Proprietor: **BRISTOL-MYERS SQUIBB COMPANY**
**Princeton, NJ 08543-4000 (US)**

(72) Inventors:
- **Scola, Paul M.**
  **Glastonbury, CT 06033 (US)**
- **Kadow, John F.**
  **Wallingford, CT 06492 (US)**
- **Vyas, Dolatrai M.**
  **Madison, CT 06443 (US)**

(74) Representative: **Kinzebach, Werner, Dr. et al**
**Patent Attorneys,**
**Reitstötter, Kinzebach & Partner,**
**Sternwartstrasse 4**
**81679 München (DE)**

(56) References cited:
**EP-A- 0 604 910** **EP-A- 0 639 577**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 747 385 B1

## Description

The present invention concerns antitumor compounds. More particularly, the invention provides novel paclitaxel derivatives, pharmaceutical formulations thereof, and their use as antitumor agents.

Taxol® (paclitaxel) is a natural product extracted from the bark of Pacific yew trees, Taxus brevifolia. It has been shown to have excellent antitumor activity in in vivo animal models, and recent studies have elucidated its unique mode of action, which involves abnormal polymerization of tubulin and disruption of mitosis. It has recently been approved for the treatment of refractory advanced ovarian cancer and breast cancer; and studies involving other cancers have shown promising results. The results of paclitaxel clinical studies are reviewed by numerous authors, such as by Rowinsky and Donehower in "The Clinical Pharmacology and Use of Antimicrotubule Agents in Cancer Chemotherapeutics," Pharmac. Ther., 52:35-84, 1991; by Spencer and Faulds in "**Paclitaxel**, A Review of its Pharmacodynamic and Pharmacokinetic Properties and Therapeutic Potential in the Treatment of Cancer," Drugs, 48 (5) 794-847, 1994; and by K.C. Nicolaou et al. in "Chemistry and Biology of Taxol," Angew, Chem., Int. Ed. Engl., 33: 15-44, 1994, and also in the references cited therein.

A semi-synthetic analog of paclitaxel named Taxotere® (docetaxel) has also been found to have good antitumor activity in animal models. Taxotere® is also currently undergoing clinical trials in Europe and in the United States. The structures of paclitaxel and Taxotere® are shown below along with the conventional numbering system for molecules belonging to the class; such numbering system is also employed in this application.

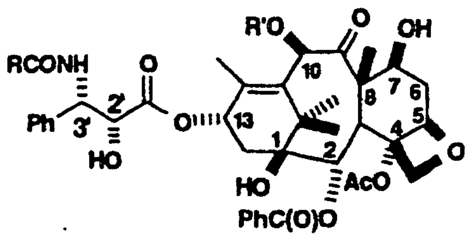

| | |
|---|---|
| Taxol®: | R = Ph; R' = acetyl |
| Taxotere®: | R = t-butoxy; R' = hydrogen |

One drawback of paclitaxel is its very limited water solubility requiring it to be formulated in nonaqueous pharmaceutical vehicles. One commonly used carrier is Cremophor® EL which may itself have undesirable side effects in man. Accordingly, a number of research teams have prepared water-soluble derivatives of paclitaxel, some of them are disclosed in the following references:

(a) Haugwitz et al, US-A-4,942,184;
(b) Kingston et al, US-A-5,059,699;
(c) Stella et al, US-A-4,960,790;
(d) European Patent Application 0,558,959 A1, published September 8, 1993;
(e) Vyas et al, Bioorganic & Medicinal Chemistry Letters, 1993, 3:1357-1360; and
(f) Nicolaou et al, Nature, 1993, 364:464-466
(g) European Patent Application EP-A-0,604,910, published July 6, 1994.

Compounds of the present invention are water soluble phosphonooxymethyl carbamate derivatives of paclitaxels and pharmaceutically acceptable salts thereof. The water solubility of the salts facilitates preparation of pharmaceutical formulations.

This invention relates to novel antitumor compounds represented by formula I, or pharmaceutically acceptable salts thereof

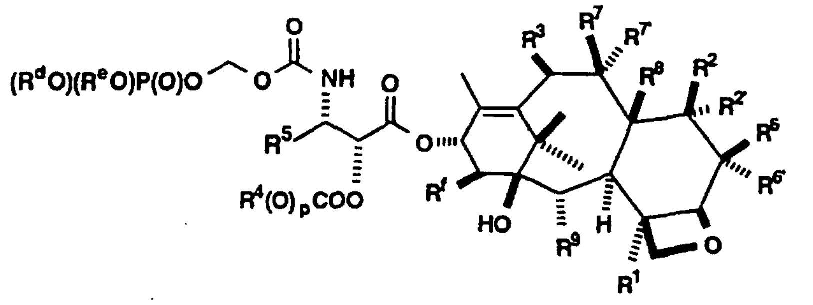

I

wherein $R^1$ is hydroxy, $-OC(O)R^x$ or $-OC(O)OR^x$; $R^2$ is hydrogen, hydroxy, $-OC(O)R^x$ or $-OC(O)OR^x$; $R^{2'}$ is hydrogen, hydroxy or fluoro; $R^{6'}$ is hydrogen or hydroxy; $R^6$ is hydrogen, or $R^2$ and $R^6$ together can form oxirane ring or a bond; $R^3$ is hydrogen, hydroxy, $C_{1-6}$ alkyloxy, $-OCONR^{11}R^{12}$, $-OC(O)R^x$ or $-OC(O)OR^x$; $R^8$ is methyl or hydroxymethyl, or $R^8$ and $R^2$ together can form cyclopropane ring; $R^9$ is hydroxy or $-OC(O)R^x$; with the proviso that when $R^8$ and $R^2$ form cyclopropane ring, $R^{2'}$ is hydrogen; when $R^2$ and $R^6$ form oxirane ring or double bond, $R^{2'}$ and $R^{6'}$ are hydrogen; when $R^2$ is hydroxy, $-OC(O)R^x$ or $-OC(O)OR^x$, $R^{2'}$ is hydrogen; when $R^{2'}$ is fluoro, $R^2$ is hydrogen; one of $R^7$ or $R^{7'}$ is hydrogen and the other is hydroxy, $-OC(O)R^x$ or $-OC(O)OR^x$, or $R^7$ and $R^{7'}$ together can form an oxo group; $R^{11}$ and $R^{12}$ are independently $C_{1-6}$ alkyl, hydrogen, aryl or substituted aryl; $R^4$ and $R^5$ are independently $C_{1-6}$ alkyl, $C_{2-6}$ alkenyl, $C_{2-6}$ alkynyl, or $-Z-R^{10}$; Z is a direct bond, $C_{1-6}$ alkyl or $C_{2-6}$ alkenyl; $R^{10}$ is aryl, substituted aryl, $C_{3-6}$ cycloalkyl or heteroaryl; p is 0 or 1; $R^d$ and $R^e$ are independently hydrogen, $C_{1-6}$ alkyl, aryl, substituted aryl or phosphono protecting group; $R^f$ is hydrogen or hydroxy; $R^x$ is $C_{3-6}$ cycloalkyl, $C_{2-6}$ alkenyl or $C_{1-6}$ alkyl, all can be optionally substituted with one to six same or different halogen atoms; or $R^x$ is a radical of the formula

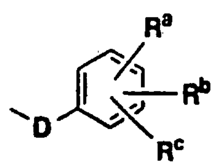

wherein D is a bond or $C_{1-6}$ alkyl; and $R^a$, $R^b$ and $R^c$ are independently hydrogen, nitro, amino, $C_{1-6}$ alkylamino, di-$C_{1-6}$ alkylamino, halogen, $C_{1-6}$ alkyl, hydroxy or $C_{1-6}$ alkoxy.

Another aspect of the present invention provides the use of a compound of formula I for preparing a pharmaceutical comparition for inhibiting tumor in a mammalian host which comprises administering to said mammalian host an antitumor effective amount of a compound of formula I.

Yet, another aspect of the present invention provides a pharmaceutical formulation which comprises an antitumor effective amount of a compound of formula I in combination with one or more pharmaceutically acceptable carriers, excipients, diluents or adjuvants.

In the application, unless otherwise specified explicitly or in context, the following definitions apply. The numbers in the subscript after the symbol "C" define the number of carbon atoms a particular group can contain. For example "$C_{1-6}$ alkyl" means a straight or branched saturated carbon chain having from one to six carbon atoms; examples include methyl, ethyl, n-propyl, isopropyl, n-butyl, sec-butyl, isobutyl, t-butyl, n-pentyl, sec-pentyl, isopentyl, and n-hexyl. Depending on the context, "$C_{1-6}$ alkyl" can also refer to $C_{1-6}$ alkylene which bridges two groups; examples include propane-1,3-diyl, butane-1,4-diyl, 2-methyl-butane-1,4-diyl, "$C_{2-6}$ alkenyl" means a straight or branched carbon chain having at least one carbon-carbon double bond, and having from two to six carbon atoms; examples include ethenyl, propenyl, isopropenyl, butenyl, isobutenyl, pentenyl, and hexenyl. Depending on the context, "$C_{2-6}$ alkenyl" can also refer to $C_{2-6}$ alkenediyl which bridges two groups; examples include ethylene-1,2-diyl (vinylene), 2-methyl-2-butene-1,4-diyl, 2-hexene-1,6-diyl, "$C_{2-6}$ alkynyl" means a straight or branched carbon chain having at least one carbon-carbon triple bond, and from two to six carbon atoms; examples include ethynyl, propynyl, butynyl, and hexynyl.

"Aryl" means aromatic hydrocarbon having from six to ten carbon atoms; examples include phenyl and naphthyl. "Substituted aryl" means aryl independently substituted with one to five (but preferably one to three) groups selected from $C_{1-6}$ alkanoyloxy, hydroxy, halogen, $C_{1-6}$ alkyl, trifluoromethyl, $C_{1-6}$ alkoxy, aryl, $C_{2-6}$ alkenyl, $C_{1-6}$ alkanoyl, nitro, amino, $C_{1-6}$ alkylamino, di-$C_{1-6}$ alkylamino, and amido. "Halogen" means fluorine, chlorine, bromine, and iodine.

"Heteroaryl" means a five- or six-membered aromatic ring containing at least one and up to four non-carbon

atoms selected from oxygen, sulfur and nitrogen. Examples of heteroaryl include thienyl, furyl, pyrrolyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, pyrazinyl, pyridazinyl, triazinyl, tetrazinyl.

"Hydroxy protecting groups" include, but is not limited to, ethers such as methyl, t-butyl, benzyl, p-methoxybenzyl, p-nitrobenzyl, allyl, trityl, methoxymethyl, methoxyethoxymethyl, ethoxyethyl, tetrahydropyranyl, tetrahydrothiopyranyl, dialkylsilylethers, such as dimethylsilyl ether, and trialkylsilyl ethers such as trimethylsilyl ether, triethylsilyl ether, and t-butyldimethylsilyl ether; esters such as benzoyl; acetyl, phenylacetyl, formyl, mono-, di-, and trihaloacetyl such as chloroacetyl, dichloroacetyl, trichloroacetyl, trifluoroacetyl; and carbonates such as methyl, ethyl, 2,2,2-trichloroethyl, allyl, benzyl, and p-nitrophenyl.

"Phosphono" means the group $-P(O)(OH)_2$ and "(phosphonooxymethyl)oxy" means $-OCH_2OP(O)(OH)_2$.

"Phosphono protecting groups" means moieties which can be employed to block or protect the phosphono functional group; preferably such protecting groups are those that can be removed by methods that do not appreciably affect the rest of the molecule. Suitable phosphonooxy protecting groups are well known to those skilled in the art and include, for example, benzyl and allyl groups.

Additional examples of hydroxy and phosphono protecting groups may be found in standard reference works such as Greene and Wuts, Protective Groups in Organic Synthesis, 2d Ed., 1991, John Wiley & Sons, and McOmie; and Protective Groups in Organic Chemistry, 1975, Plenum Press. Methods for introducing and removing protecting groups are also found in such textbooks.

"Pharmaceutically acceptable salt" means a metal or an amine salt of the acidic phosphono group in which the cation does not contribute significantly to the toxicity or biological activity of the active compound. Suitable metal salts include lithium, sodium, potassium, calcium, barium, magnesium, zinc, and aluminum salts. Preferred metal salts are sodium and potassium salts. Suitable amine salts are for example, ammonia, tromethamine (TRIS), triethylamine, procaine, benzathine, dibenzylamine, chloroprocaine, choline, diethanolamine, triethanolamine, ethylenediamine, glucamine, N-methylglucamine, lysine, arginine, ethanolamine, to name but a few.

The term "taxane" or "taxane core" refers to moieties with frameworks of the structure:

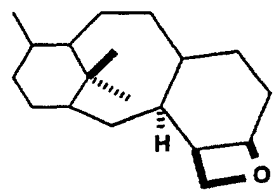

The cycloprane group which can be constituted from $R^8$ and $R^2$ of formula I can alternatively be referred to as "7β,8β-methano" group as in Tetrahedron Letters, Vol 35, No 43, pp 7893-7896 (1994) or as "cyclopropa" group as in US-A-5,254,580 issued October 19, 1993. When $R^2$ and $R^6$ form a bond, naturally there will be a double bond between C7 and C6.

In compounds of formula I, examples of $R^x$ include methyl, hydroxymethyl, ethyl, n-propyl, isopropyl, n-butyl, isobutyl, chloromethyl, 2,2,2-trichloroethyl, cyclopropyl, cyclobutyl, cyclopentyl, cyclohexyl, ethenyl, 2-propenyl, phenyl, benzyl, bromophenyl, 4-aminophenyl, 4-methylaminophenyl, 4-methylphenyl, 4-methoxyphenyl. Examples of $R^4$ and $R^5$ include 2-propenyl, isobutenyl, 3-furanyl (3-furyl), 3-thienyl, phenyl, naphthyl, 4-hydroxyphenyl, 4-methoxyphenyl, 4-fluorophenyl, 4-trifluoromethylphenyl, methyl, ethyl, n-propyl, isopropyl, n-butyl, isobutyl, t-butyl, ethenyl, 2-propenyl, 2-propynyl, benzyl, phenethyl, phenylethenyl, 3,4-dimethoxyphenyl, 2-furanyl (2-furyl), 2-thienyl, 2-(2-furanyl) ethenyl, 2-methylpropyl, cyclopropyl, cyclobutyl, cyclopentyl, cyclohexyl, cyclohexylmethyl, cyclohexylethyl.

The new products that have the general formula I display a significant inhibitory effect with regard to abnormal cell proliferation, and have therapeutic properties that make it possible to treat patients who have pathological conditions associated with an abnormal cell proliferation. The pathological conditions include the abnormal cellular proliferation of malignant or non-malignant cells in various tissues and/or organs, including, non-limitatively, muscle, bone and/or conjunctive tissues; the skin, brain, lungs and sexual organs; the lymphatic and/or renal system; mammary cells and/or blood cells; the liver, digestive system, and pancreas; and the thyroid and/or adrenal glands. These pathological conditions can also include psoriasis; solid tumors; ovarian, breast, brain, prostate, colon, stomach, kidney, and/or testicular cancer, Karposi's sarcoma; cholangiocarcinoma; choriocarcinoma; neuroblastoma; Wilm's tumor, Hodgkin's disease; melanomas; multiple myelomas; chronic lymphocytic leukemias; and acute or chronic granulocytic lymphomas. The novel products in accordance with the invention are particularly useful in the treatment of non-Hodgkin's lymphoma, multiple myeloma, melanoma, and ovarian, urothelial, oesophageal, lung, and breast cancers. The products in accordance with the invention can be utilized to prevent or delay the appearance or reappearance, or to treat these

pathological conditions.

The compounds of this invention can be made by techniques from the conventional organic chemistry repertoire. Scheme I, which depicts a process that compounds within the scope of formula I can be made, is only shown for the purpose of illustration.

The "acid" in a compound of formula II is any acid which is able to protonate the C'3-amino group. Exemplary acidic salts include salts formed with mineral acids such as HCl, $H_2SO_4$, or $HNO_3$; or organic acids such as trifluoroacetic acid, acetic acid, p-toluenesulfonic acid, methanesulfonic acid. Step (a) involves liberating C3'-amino group with base, followed by reacting the liberated amino group with a compound of formula III, in which $R^{13}$ and $R^{14}$ are independently $C_{1-6}$ alkyl, aryl, substituted aryl or phosphono protecting group. The base can be any base to neutralize the amino protonating acid and which also acts as an acceptor of proton generated during the reaction of amine with chloroformate of formula III. Examples of preferable base include inorganic base or organic base such as diisopropylethylamine, triethylamine, pyridine, dimethylaminopyridine.

When $R^{13}$ and/or $R^{14}$ is phosphono protecting group, it is removed in Step (b) to afford additional compounds withing the scope of formula I.

SCHEME I

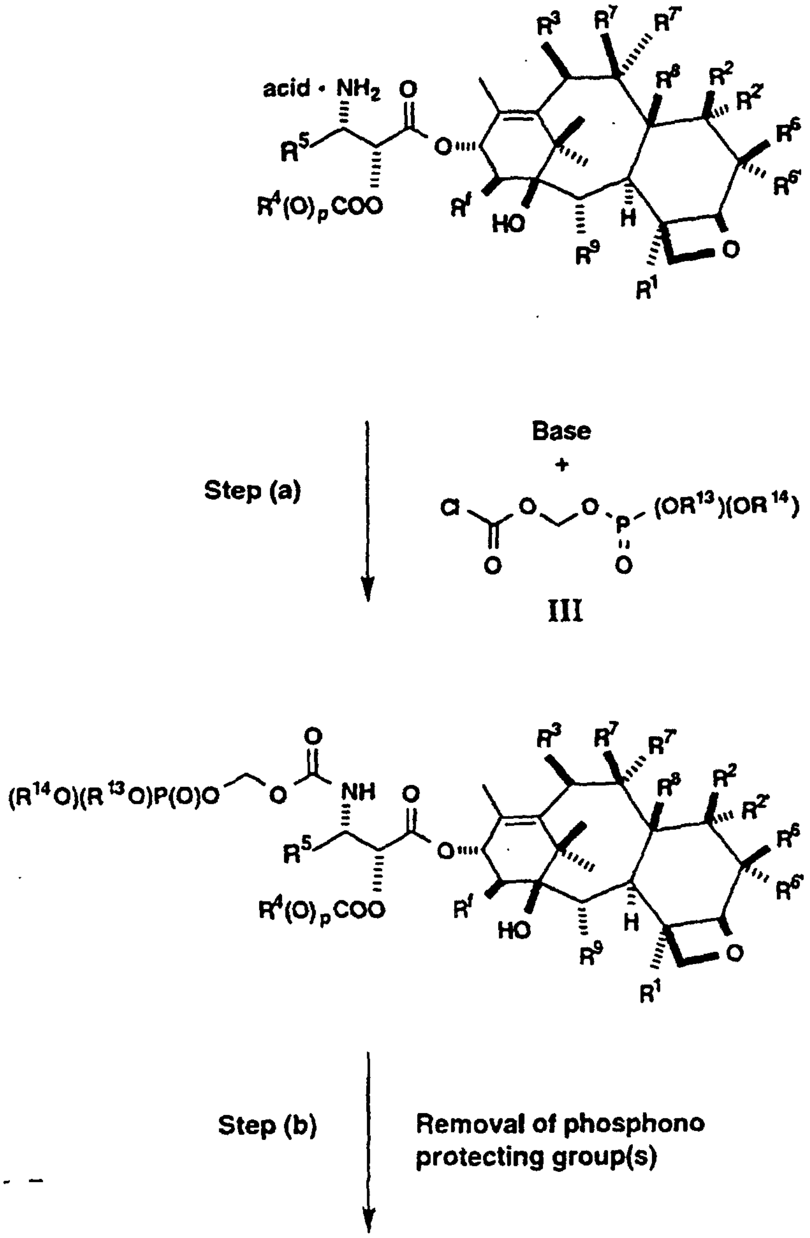


II

III

I'

I''

The synthesis of compounds of formula II is well delineated in our PCT application WO 94/14787 published July 7, 1994. Briefly, they are made by steps comprising:

(a) coupling the oxazoline of the formula V

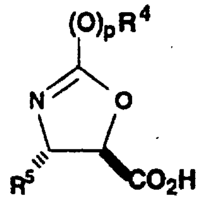

V

with C13-hydroxy of taxane of formula VI

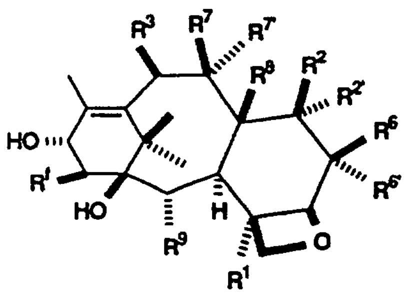

VI

to afford a compound of formula VII

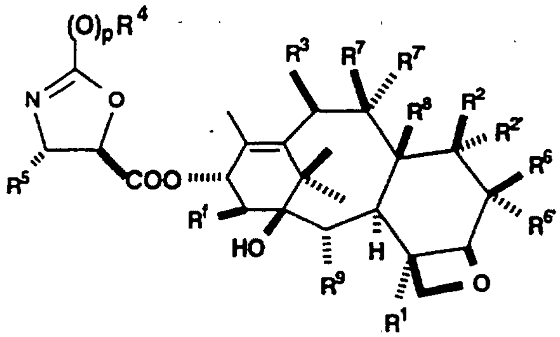

VII ;

and

(b) contacting a compound of formula VII with an acid capable of opening the oxazoline ring of said compound of the formula VII to afford a compound of formula II or salt thereof.

Oxazolines of formula V are already well described in our PCT application WO 94/14787 published July 7, 1994.

In formulas II, III, V, VI and VII above, p, $R^f$, $R^1$, $R^2$, $R^{2'}$, $R^3$, $R^4$, $R^5$, R6, $R^{6'}$, $R^7$, $R^{7'}$, $R^8$ and $R^9$ are as previously defined;

By now there are many publications teaching the conversion of paclitaxel taxane core substitutents into other groups. Using these established methods or obvious variants thereof, taxanes of formula VI can be readily made. For example, for transforming C4-acetoxy into other functional groups see, S. H. Chen et al., *J. Organic Chemistry,* 59, pp 6156-6158 (1994) and PCT application WO 94/14787 published July 7, 1994; for converting C2-benzoyloxy to other groups see, S.H. Chen et al, *Bioorganic and Medicinal Chemistry Letters*, Vol. 4, No. 3, pp 479-482 (1994) and European Patent Application EP-A-617,034 published September 28, 1994; for modifying C10-acetyloxy see, J. Kant et al, *Tetrahedron Letters,* Vol. 35, No. 31, pp 5543-5546 (1994) and US-A-5,294,637 issued March 15, 1994; for making C10 and/or C7 unsubstituted (deoxy) derivatives see, European Patent Application EP-A-590,267 published April 6, 1994 and PCT application WO 93/06093 published April 1, 1993; for making 7β,8β-methano, 6α,7α-dihydroxy and 6,7-olefinic groups see, R. A. Johnson, *Tetrahedron Letters,* Vol. 35, No 43, pp 7893-7896 (1994), U.S. Patent No. 5,254,580 issued October 19, 1993, and European Patent Application 600,517A1 published June 8, 1994; for making C7/C6

oxirane see, X. Liang and G.I. Kingston, *Tetrahedron Letters,* Vol. 36, No. 17, pp 2901-2904 (1995); for making C7-epi-fluoro see, G. Roth et al, *Tetrahedron Letters,* Vol 36, pp 1609-1612 (1993); for forming C7 esters and carbonates see, US-A-5,272,171 issued December 21, 1993 and S. H. Chen et al., *Tetrahedron,* 49, No. 14, pp 2805-2828 (1993); for 9α- and 9β-hydroxy taxanes see, L. L. Klein, *Tetrahedron Letters,* Vol 34, No 13, pp 2047-2050 (1993), PCT application WO 94/08984 published April 28, 1994, US-A 5,352,806 issued October 4, 1994, and PCT application WO 94/20485 published September 15, 1994.

DESCRIPTION OF SPECIFIC EMBODIMENTS

The specific examples that follow illustrate the syntheses of the compound of the instant invention The method may be adapted to variations in order to produce the compound embraced by this invention but not specifically disclosed. Further, variations of the methods to produce the same compound in somewhat different manner will also be evident to one skilled in the art.

In the following experimental procedures, all temperatures are understood to be in Centigrade (C) when not specified. The nuclear magnetic resonance (NMR) spectral characteristics refer to chemical shifts (δ) expressed in parts per million (ppm) versus tetramethylsilane (TMS) as reference standard. The relative area reported for the various shifts in the proton NMR spectral data corresponds to the number of hydrogen atoms of a particular functional type in the molecule. The nature of the shifts as to multiplicity is reported as broad singlet (bs or br s), broad doublet (bd or br d), broad triplet (bt or br t), broad quartet (bq or br q), singlet (s), multiplet (m), doublet (d), quartet (q), triplet (t), doublet of doublet (dd), doublet of triplet (dt), and doublet of quartet (dq). The solvents employed for taking NMR spectra are acetone-$d_6$ (deuterated acetone). DMSO-$d_6$ (perdeuterodimethylsulfoxide), $D_2O$ (deuterated water), $CDCl_3$ (deuterochloroform) and other conventional deuterated solvents. The infrared (IR) spectral description include only absorption wave numbers ($cm^{-1}$) having functional group identification value.

Celite® is a registered trademark of the Johns-Manville Products Corporation for diatomaceous earth.

The abbreviations used herein are conventional abbreviations widely employed in the art. Some of which are: DAB (deacetylbaccatin III); MS (mass spectrometry); HRMS (high resolution mass spectrometry); Ac (acetyl); Ph (phenyl); v/v (volume/volume); FAB (fast atom bombardment); NOBA (m-nitrobenzyl alcohol); min (minute(s)); h or hr(s) (hour(s)); BOC (t-butoxycarbonyl); CBZ or Cbz (benzyloxycarbonyl); Bn (benzyl); Bz (benzoyl); Troc (2,2,2-trichloroethyloxycarbonyl), DMS (dimethylsilyl), TBAF (tetrabutylammonium fluoride), DMAP (4-dimethylaminopyridine); TES (triethylsilyl); DMSO (dimethylsulfoxide); THF (tetrahydrofuran); HMDS (hexamethyldisilazane); MeOTf (methyltriflate).

1. Preparation of O-chloromethyl-S-butylcarbonothioate.

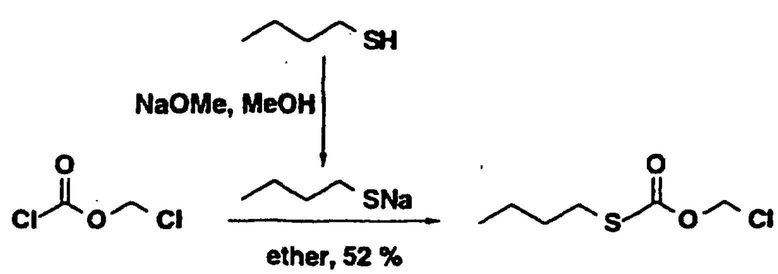


Preparation of O-Chloromethyl-S-butylcarbonothioate was followed according to the method described by Folkman, M. and Lund, F. in *Synthesis ,* **1990**, 1159. Butane thiol (16.0 mL, 200 mmol) was added dropwise to a solution of sodium methoxide in methanol (43 mL, 200 mmol, 25% by wt., Aldrich) cooled to 0°C and the resulting mixture was stirred for 2 h. The reaction mixture was then concentrated in vacuo and the residual solid suspended in anhydrous ethyl ether (300 mL). This heterogenous solution was then cooled to -78°C and a solution of chloromethyl chloroformate (17.6 mL, 200 mmol) in ether (70 mL) was added dropwise over 40 min. The resulting solution was stirred at -78°C for an additional 2 h, and the reaction mixture was then warmed to room temperature and stirred for 13 h. The reaction mixture was then suction filtered using a pad of Celite®, the collected salts were washed with ether, and the filtrate concentrated in vacuo. The resulting residual oil was purified via fractional distillation (bp 103-107°C, house vaccum approximately 25-30 mbarr, lit. bp 99-101°C, 24 mbarr); a center cut provided the desired O-chloromethyl S-butylcarbonothioate (18 g, 52%). $^1H$ NMR (300 MHz, $CDCl_3$) δ 5.72 (2H, s), 2.90 (2H, dd, J = 8.7,8.7 Hz), 1.63-1.51 (2H, m), 1.45-1.31 (2H, m), 0.91-0.82 (3H, m).

2. Preparation of O-iodomethyl-S-butylcarbonothioate

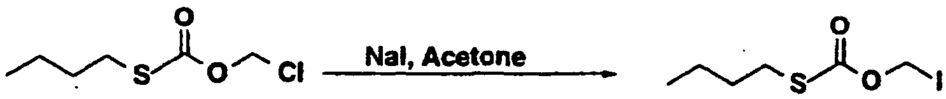


A solution of O-chloromethyl-S-butylcarbonothioate (10.0 g, 0.054 mol) in acetone (10 mL) was added to a solution of sodium iodide (16.4 g, 0.108 mol, 2 eqiuv.) and sodium bicarbonate (0.461 g, 0.0054 mol, 0.1 equiv.) in acetone (200 mL) at room temperature. The reaction mixture was then warmed to 45°C and stirred for 2h. At this time an aliquot of the reaction mixture was concentrated in vacuo and the residual oil examined by $^1$H NMR which indicated consumption of starting material and formation of a single product. The remaining reaction mixture was then filtered using a pad of Celite® and the filtrate concentrated in vacuo. The residual oil was then partitioned between water and pentane and the organic layer was further washed with aqueous solutions of 5% sodium bicarbonate, 1% sodium thiosulfate and brine. The aqueous layers were back extracted with pentane and the combined organics were dried over sodium sulfate and concentrated in vacuo. The resulting residual oil was cleaned by $^1$H NMR analysis and the desired O-iodomethyl-S-butylcarbonothioate was used without further purification. $^1$H NMR (300 MHz, $CDCl_3$) δ 5.93 (2H, s, downfield shift from corresponding chloro compound), 2.83 (2H, dd, J = 8.7,8.7 Hz), 1.62-1.51 (2H, m), 1.48-1.36 (2H, m), 0.92-0.84 (3H, m).

3. Preparation of tetrabutylammonium dibenzylphosphate salt

$$(BnO)_2P(O)OH + HON(Bu)_4 \rightarrow (BnO)_2P(O)ON(Bu)_4$$

Dibenzylphosphate (15.0 g, 0.054 mol) was added to a solution of tetrabutylammonium hydroxide (40% wt. in water, Aldrich, 35.0 g, 0.054 mol) in water at room temperature and the resulting homogeneous solution was cooled in a dry-ice acetone bath. until solidification was complete. Removal of water via lyophilization provided the desired salt as a viscous oil which was used without further purification.

4. Preparation of O-dibenzylphosphonooxymethyl-S-butylcarbonothioate

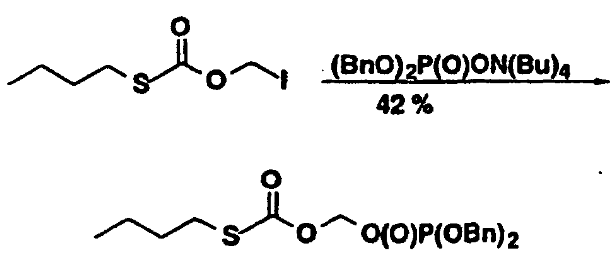


A solution of O-iodomethyl-S-butylcarbonothioate (crude, 0.054 mol) in THF (20 mL) was added to a solution of tetrabutylammonium dibenzylphosphate (28.5 g, 0.054 mol) in THF (150 mL) at room temperature and the resulting mixture was stirred for 24 h. The reaction mixture was then filtered using a pad of Celite® and the filtrate was concentrated in vacuo. The residual oil was purified via flash chromatography (eluted with hexanes/ethyl acetate) from which a center cut provided the desired O-dibenzylphosphonooxymethyl-S-butylcarbonothioate (10.0 g, 42.5%) as light yellow oil. $^1$H NMR (300 MHz, $CDCl_3$) δ 7.34 (10H, brs), 5.62 (2H, d, J = 14.0 Hz), 5.05 (4H, d, J = 7.8 Hz), 2.82 (2H, dd, J = 7.3 Hz), 1.62-1.51 (2H, m), 1.41-1.30 (2H, m), 0.89 (3H, dd, J = 9.4 Hz).

5. Preparation of O-dibenzylphosphonooxymethyl chloroformate

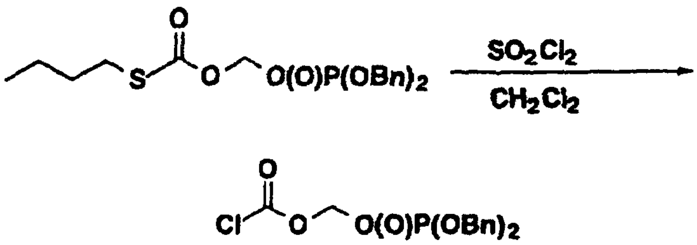


Distilled sulfuryl chloride (1.29 mL, 0.0160 mol, 1.2 eq.) was added in one portion to a solution of the O-dibenzylphosphonooxymethyl-S-butylcarbonothioate (5.7 g, 0.0134 mol, 1.0 eq.) in dichloromethane cooled to -40°C. The reaction mixture was stirred at this temperature for 20 min at which time the cooling bath was removed and the reaction mixture warmed to room temperature and stirred for 3 h. The reaction mixture was then concentrated in vacuo and the residual oil subject to high vacuum to remove by-products of the reaction and any remaining sulfuryl chloride. $^1H$ NMR analysis of the crude chloroformate indicated approximately 60% conversion of the starting material O-dibenzylphosphonooxymethyl-S-butylcarbonothioate to the corresponding chloroformate which was used without further purification in the following transformation. $^1H$ NMR, Selected resonances of chloroformate (300 MHz, $CDCl_3$) δ 5.59 (2H, d, J = 14.0 Hz, upfield shilft relative to corresponding thiocarbonate), 5.10 (4H, d, J = 7.8 Hz, downfield shift relative to corresponding thiocarbonate).

6. Preparation of N-debenzoyl-N-[[(dibenzylphosphonooxy)methyl]oxy]carbonyl-2-O'-benzoylpaclitaxel (Ia)

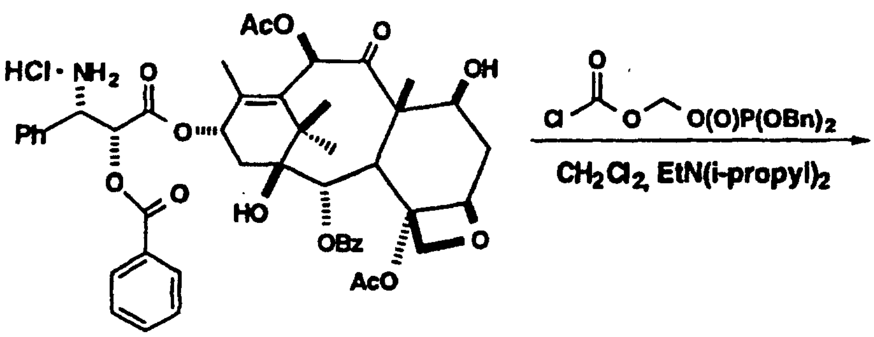


IIa

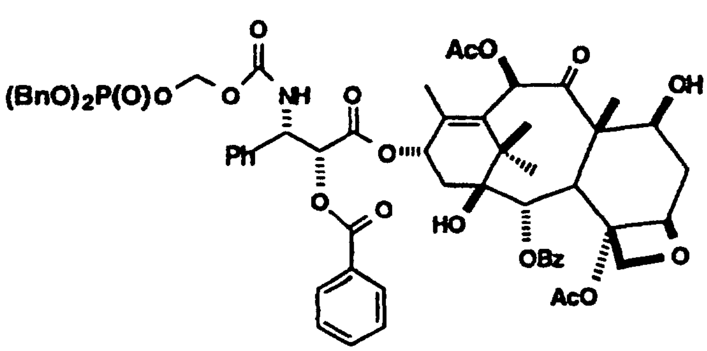

Ia

To a solution of the chloroformate (ca. 0.0080 mol, 1.7 eq. at 60% conversion) in dichloromethane cooled to 0°C was added diisopropylethyl amine (4.7 mL, 0.0208 mol, 5.eq) followed by N-debenzoyl-2-Q'-benzoylpaclitaxel hydrochloride (IIa) (4.0 g, 0.00449 mmol). Additional diisopropylethyl amine (4.7 mL, 0.0208 mol) was then added, the cooling bath was removed, and the reaction mixture was warmed to room temperature and stirred for an additional 1.5 h. The reaction mixture was then diluted with ethyl acetate and quenched by the addition of saturated aqueous sodium bicarbonate. The organic layer was then removed and washed with a saturated aqueous ammonium chloride solution followed by brine. The aqueous layers were then back extracted with ethyl acetate and the combined organics were dried over sodium sulfate and concentrated in vacuo to provide a light yellow oily solid. Purification of the crude solid via flash chromatography (eluted with hexane/ethyl acetate) provided 2.9 g (55%) of the desired dibenzylphosphate as a white solid. $^1H$ NMR (300 MHz, $CDCl_3$) δ 8.19-8.11 (2H, m), 7.98-7.89 (2H, m), 7.61-7.18 (21H, m), 7.10-7.07 (1H, m), 6.43 (1H, dd, J = 8.6, 8.6 Hz), 6.12 (1H, d, J = 9.9 Hz), 5.78-5.61 (4H, m), 5.18 (1H, dd, J = 5.2 Hz, 14.2 Hz), 5.01-4.92 (2H, m), 4.75-4.55 (2H, m), 4.51-4.42 (1H, m), 4.31-4.27 (2H, m), 3.86 (1H, d, J = 7.2 Hz), 3.62 (1H, brs), 2.67-1.65 (16H, m, including singlets at 2.51, 2.23, 2.01, and 1.22, 3H each), 1.21 (3H, s), 1.15 (3H, s); Mass Spec. ($M+Na^+$) 1210.

7. Preparation of N-debenzoyl-N-[(phosphonooxymethyl)oxy]carbonyl-2'-O-benzoylpaclitaxel (Ib)

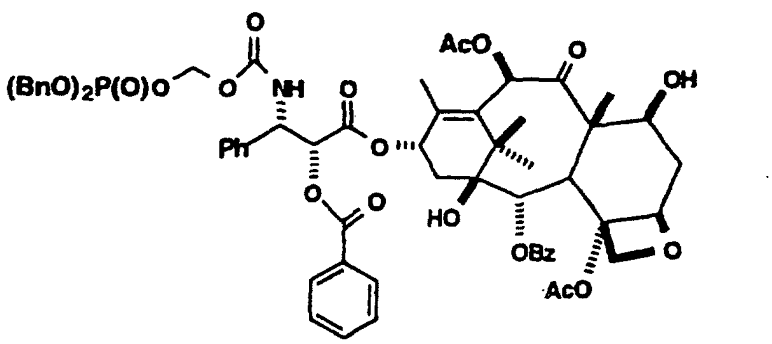

Ia

1. $H_2$, EtOAc
2. $N(EtOH)_3$, EtOAc, MeOH
3. C18 Chrom.

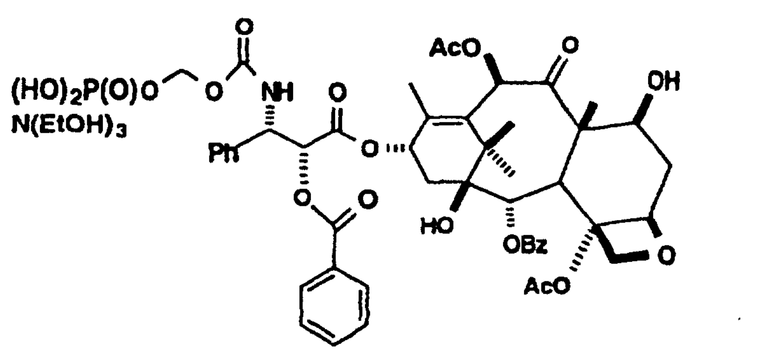

Ib

Ethyl acetate (200 mL) was added to 10% palladium on carbon (3.0 g) in a parr hydrogenation vessel. A

solution of N-debenzoyl-N-[[(dibenzylphosphonooxy)methyl]oxy]carbonyl-2'-O-benzoylpaclitaxel (Ia) (2.9 g, 0.0024 mol) in ethyl acetate (50 mL) was then added and the reaction vessel was affixed to a parr hydrogenation apparatus. The reaction mixture was then evacuated for approximately 1 min using house vacuum and subsequently pressurized with hydrogen gas to 3,5·$10^5$ Pa (50 psi) This proceedure was repeated three times after which the reaction vessel was maintained at 3,5·$10^5$ Pa (50 psi) and shaken for 12 h. The reaction mixture was then filtered using a sintered glass funnel (fine porousity) and during this process methanol (approximately 50 mL) was added to completely dissolve the phosphate free acid and facilitate the filtration process. An aliqout of the filtrate containing the prodrug was concentrated in vacuo and analyzed on HPLC (85% purity observed). To the remaining filtrate was then added a solution of triethanol amine in ethyl acetate (0.1 M, 23 mL, 0.0023 mol, 0.95 eq.) and the resulting solution was concentrated in vacuo. The crude prodrug salt was purified via medium pressure C18 chromatography. In this process the crude phosphate amine salt was taken-up as a suspension in 5% acetonitrile in water (approximately 50-80 mL) and applied to the C18 column (equilibrated with 5% acetonitrile in water). A gradient elution technique was employed (5% acetonitrile : 95% water, 10%:90%, 15%:85%, 20%:80%, 25%:75%, 30%:70%) and fractions containing compound Ib (> 95% purity by HPLC) were combined and concentrated in vacuo to remove acetonitrile. The remaining aqueous solution of compound Ib was then frozen and water removed via lyophilization to provide 1.34 g (51%) of compound Ib as a light, white solid. $^1H$ NMR (300 MHz,$CD_3OD$ : $CDCl_3$ appox. 2:1, v/v) δ 8.11-8.02 (4H, m), 7.66-7.35 (11H, m), 7.24 (1H, dd, J = 7.2, 7.2 Hz), 6.24 (1H, dd, J = 8.7,8.7 Hz), 5.65-5.43 (5H, m), 4.97 (1H, d, J = 8.4 Hz), 4.37 (1H, dd, J = 6.5, 10.7 Hz), 4.25-4.19 (2H, m), 3.85-3.79 (7H, m), 3.33-3.29 (6H, m), 2.54-1.66 (16H, m, including singlets at 2.49, 2.16, 1.97, and 1.66, 3H each), 1.18 (3H, s), 1.13 (3H, s); Mass Spec. (M-1) 1006 (consistent).

Following substantially the procedures described herein, the following compounds within the scope of this invention, can be synthesized.

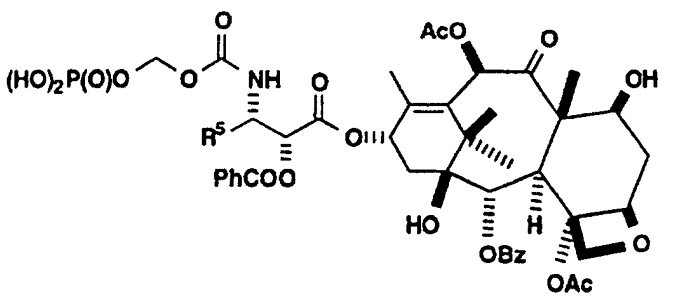

| $R^5$ |
|---|
| 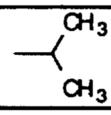 |
| 3-furyl |
| 2-furyl |
| p-fluorophenyl |
| p-chlorophenyl |
| p-methylphenyl |
| p-methoxyphenyl |
| p-bromophenyl |
| p-hydroxyphenyl |
| p-aminophenyl |
| p-nitrophenyl |
| 2-thienyl |
| 3-thienyl |
| cyclohexyl |
| cyclopentyl |
| cyclobutyl |
| cyclopropyl |
| isobutenyl |
| isopropyl |
| isobutyl |

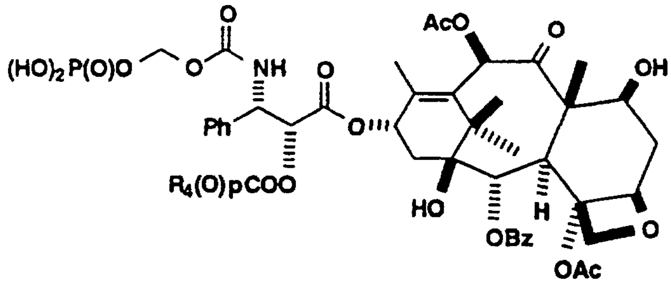

| $R4(O)_p$ |
|---|
| 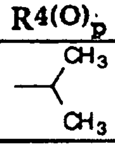 |
| 3-furyl |
| 2-furyl |
| p-fluorophenyl |
| p-chlorophenyl |
| p-methylphenyl |
| p-methoxyphenyl |
| p-bromophenyl |
| p-hydroxyphenyl |
| p-aminophenyl |
| p-nitrophenyl |
| 2-thienyl |
| 3-thienyl |
| cyclohexyl |
| cyclopentyl |
| cyclobutyl |
| cyclopropyl |
| isobutenyl |
| isopropyl |
| isobutyl |

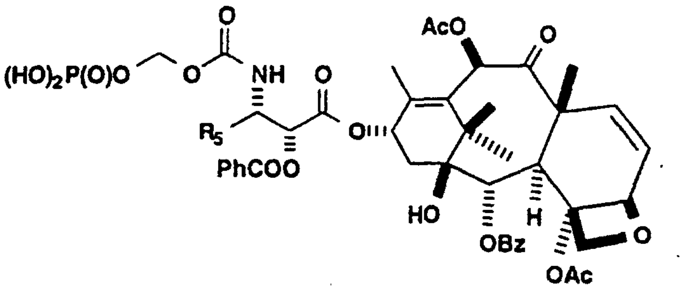

| R5 |
|---|
| 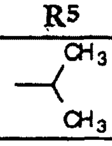 |
| 3-furyl |
| 2-furyl |
| p-fluorophenyl |
| p-chlorophenyl |
| p-methylphenyl |
| p-methoxyphenyl |
| p-bromophenyl |
| p-hydroxyphenyl |
| p-aminophenyl |
| p-nitrophenyl |
| 2-thienyl |
| 3-thienyl |
| cyclohexyl |
| cyclopentyl |
| cyclobutyl |
| cyclopropyl |
| isobutenyl |
| isopropyl |
| isobutyl |

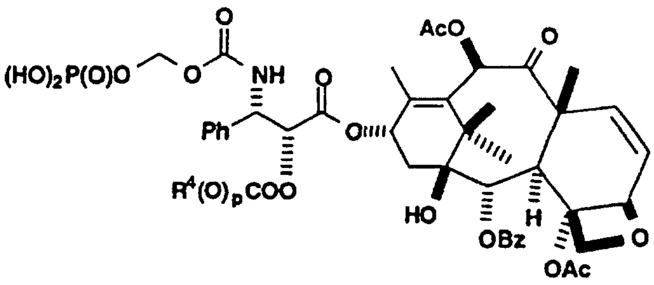

| $R^4(O)_p$ |
|---|
| 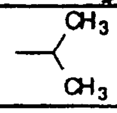 |
| 3-furyl |
| 2-furyl |
| p-fluorophenyl |
| p-chlorophenyl |
| p-methylphenyl |
| p-methoxyphenyl |
| p-bromophenyl |
| p-hydroxyphenyl |
| p-aminophenyl |
| p-nitrophenyl |
| 2-thienyl |
| 3-thienyl |
| cyclohexyl |
| cyclopentyl |
| cyclobutyl |
| cyclopropyl |
| isobutenyl |
| isopropyl |
| isobutyl |

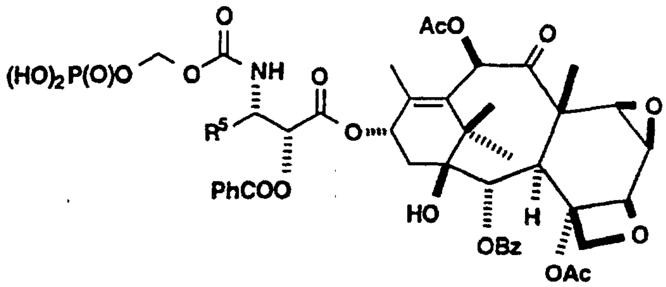

| $R^5$ |
| --- |
| 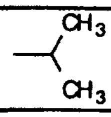 |
| 3-furyl |
| 2-furyl |
| p-fluorophenyl |
| p-chlorophenyl |
| p-methylphenyl |
| p-methoxyphenyl |
| p-bromophenyl |
| p-hydroxyphenyl |
| p-aminophenyl |
| p-nitrophenyl |
| 2-thienyl |
| 3-thienyl |
| cyclohexyl |
| cyclopentyl |
| cyclobutyl |
| cyclopropyl |
| isobutenyl |
| isopropyl |
| isobutyl |

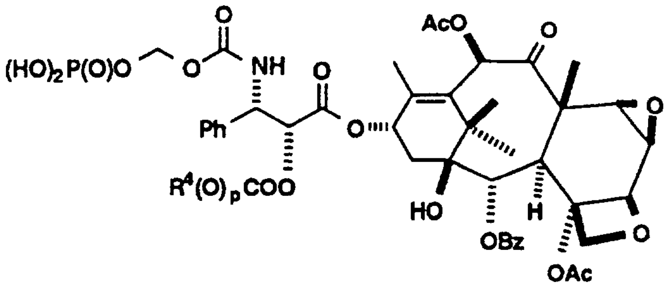

| $R^4(O)_p$ |
|---|
| 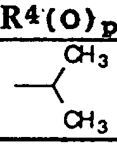 |
| 3-furyl |
| 2-furyl |
| p-fluorophenyl |
| p-chlorophenyl |
| p-methylphenyl |
| p-methoxyphenyl |
| p-bromophenyl |
| p-hydroxyphenyl |
| p-aminophenyl |
| p-nitrophenyl |
| 2-thienyl |
| 3-thienyl |
| cyclohexyl |
| cyclopentyl |
| cyclobutyl |
| cyclopropyl |
| isobutenyl |
| isopropyl |
| isobutyl |

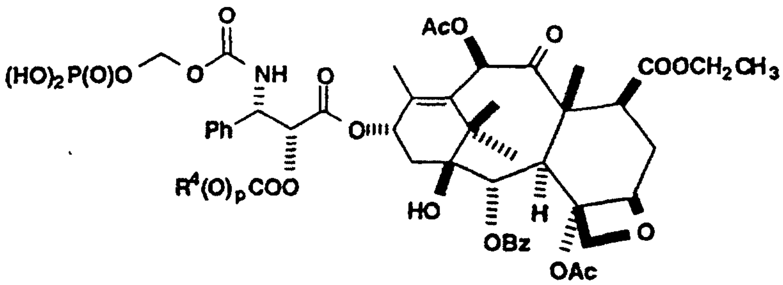

| $R^4(O)_p$ |
|---|
| 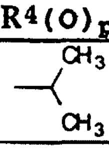 |
| 3-furyl |
| 2-furyl |
| p-fluorophenyl |
| p-chlorophenyl |
| p-methylphenyl |
| p-methoxyphenyl |
| p-bromophenyl |
| p-hydroxyphenyl |
| p-aminophenyl |
| p-nitrophenyl |
| 2-thienyl |
| 3-thienyl |
| cyclohexyl |
| cyclopentyl |
| cyclobutyl |
| cyclopropyl |
| isobutenyl |
| isopropyl |
| isobutyl |

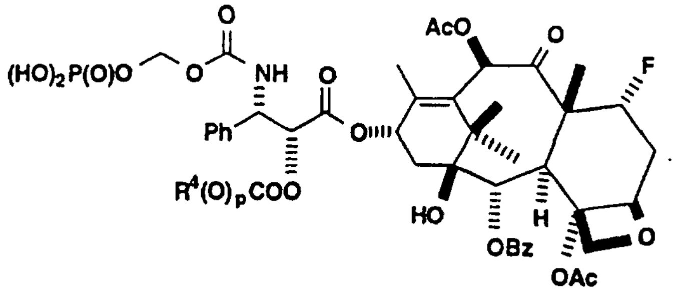

| $R^4(O)_p$ |
|---|
| 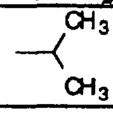 |
| 3-furyl |
| 2-furyl |
| p-fluorophenyl |
| p-chlorophenyl |
| p-methylphenyl |
| p-methoxyphenyl |
| p-bromophenyl |
| p-hydroxyphenyl |
| p-aminophenyl |
| p-nitrophenyl |
| 2-thienyl |
| 3-thienyl |
| cyclohexyl |
| cyclopentyl |
| cyclobutyl |
| cyclopropyl |
| isobutenyl |
| isopropyl |
| isobutyl |

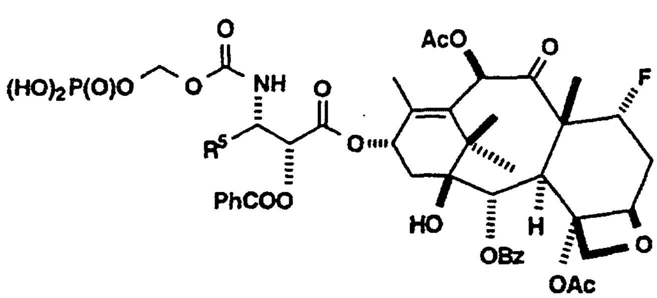

| $R^5$ |
|---|
| 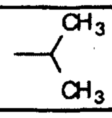 |
| 3-furyl |
| 2-furyl |
| p-fluorophenyl |
| p-chlorophenyl |
| p-methylphenyl |
| p-methoxyphenyl |
| p-bromophenyl |
| p-hydroxyphenyl |
| p-aminophenyl |
| p-nitrophenyl |
| 2-thienyl |
| 3-thienyl |
| cyclohexyl |
| cyclopentyl |
| cyclobutyl |
| cyclopropyl |
| isobutenyl |
| isopropyl |
| isobutyl |

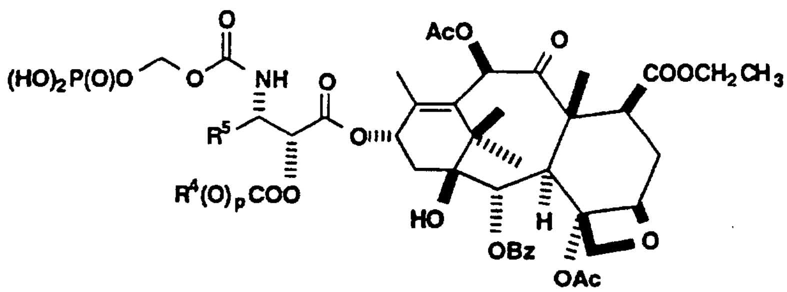

| R5 |
|---|
| 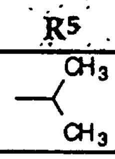 |
| 3-furyl |
| 2-furyl |
| p-fluorophenyl |
| p-chlorophenyl |
| p-methylphenyl |
| p-methoxyphenyl |
| p-bromophenyl |
| p-hydroxyphenyl |
| p-aminophenyl |
| p-nitrophenyl |
| 2-thienyl |
| 3-thienyl |
| cyclohexyl |
| cyclopentyl |
| cyclobutyl |
| cyclopropyl |
| isobutenyl |
| isopropyl |
| isobutyl |

The compound of formula I of the instant invention is an effective tumor inhibiting agent, and is useful in human and/or veterinary medicine. For example, they are effective in treating tumors in an in vivo assay described in the EP Patent Application 604,910 A1 published July 6, 1994. In one test, Balb/c x $DBA_2$ $F_1$ ($CDF_1$) hybrid mice were implanted subcutaneously (sc) with 0.1 ml of a 2% (w/v) brei of M109 lung carcinoma (as described in W. Rose "Evaluation of Madison 109 Lung Carcinoma as a Model for Screening Antitumor Drugs," Cancer Treatment Reports, 65, No. 3-4, pp. 299-312 (1981)). The test compounds and reference drug, paclitaxel, are administered intravenously to groups of mice; each group receive a compound at a different dose level, and three or four different dose levels are evaluated per compound. Additionally, the test compounds are similarly evaluated orally.

Mice are followed daily for survival until their death or about day 90 post-tumor implant, whichever occurs first One group of mice per experiment remain untreated and serve as the control. Tumors are also measured once or twice weekly and the size in mm is used to estimate tumor weight according to the published procedure (ibid). Median survival times of compound-treated (T) mice are compared to the median survival time of parallel control (C) mice. The ratio of the two values for each compound-treated group of mice is multiplied by 100 and expressed as a percentage (i.e., % T/C). Additionally, the difference between the median time for treated groups and that for the control group to grow tumor to 1 g (gm), expressed as T-C values in days, is also determined. The greater the T-C value, the greater the delay in primary tumor growth. Compounds showing % T/C ≥ 125% and/or T-C ≥ 4.0 days are considered to be active in this M109 SC model.

Compound Ib (as triethanolamine salt) was evaluated according the above protocol. In one test, when given iv in the dose range of 20-45mg/kg/inj, given once daily for 5 days beginning on day 4 post tumor implant, it had T/C values of 132 to 145% and T-C values of 8.8 to 14.0. When the compound was given orally between 200-400mg/kg/adm given once daily for 5 days beginning on day 4 post tumor implant, it had T/C values 132 to 179% and T-C values of 10.5 to 24.3 days.

Thus, another aspect of the instant invention concerns a method for inhibiting human and/or other mammalian tumors which comprises administering to a tumor bearing host an antitumor effective amount of compound of formula I.

For treating a variety of tumors, the compound of formula I of the present invention may be used in a manner similar to that of paclitaxel, e.g. see Physician's Desk Reference, 49th Edition, Medical Economics, p 682, 1995. The dosage, mode and schedule of administration for the compound of this invention are not particularly restricted; an oncologist skilled in the art of cancer treatment will be able to ascertain, without undue experimentation, an appropriate treatment protocol for administering the compound of the present invention. Thus the compound of formula I may be administered via any suitable route of administration, parenterally or orally. Parenteral administration includes intravenous, intraperitoneal, intramuscular, and subcutaneous administration.

The doses utilized to implement the methods in accordance with the invention are the ones that make it possible to administer prophylactic treatment or to evoke a maximal therapeutic response. The doses vary, depending on the type of administration, the particular product selected, and the personal characteristics of the subject to be treated. In general, the doses are the ones that are therapeutically effective for the treatment of disorders caused by abnormal cell proliferation. The products in accordance with the invention can be administered as often as necessary in order to obtain the desired therapeutic effect. Some patients may respond rapidly to relatively high or low doses, and then require mild maintenance or no maintenance dose at all. Via the iv route, the dosage may be, for example, in the range of about 20 to about 500 $mg/m^2$ over 1 to 100 hours. Via the oral route, the dosage may be in the range of 5-1000mg/kg/day of body weight. The actual dose used will vary according to the particular composition formulated, the route of administration, and the particular site, host and type of tumor being treated. Many factors that modify the action of the drug will be taken into account in determining the dosage including age, weight, sex, diet and the physical condition of the patient.

The present invention also provides pharmaceutical formulations (compositions) containing an antitumor effective amount of compound of formula I in combination with one or more pharmaceutically acceptable carriers, excipients, diluents or adjuvants. The compositions can be prepared in accordance with conventional methods. Examples of formulating paclitaxel or derivatives thereof may be found in, for example, United States Patents Nos. 4,960,790 and 4,814,470, and such examples may be followed to formulate the compound of this invention. For example, compound of formula I may be formulated in the form of tablets, pills, powder mixtures, capsules, injectables, solutions, suppositories, emulsions, dispersions, food premix, and in other suitable forms. It may also be manufactured in the form of sterile solid compositions, for example, freeze dried and, if desired, combined with other pharmaceutically acceptable excipients. Such solid compositions can be reconstituted with sterile water, physiological saline, or a mixture of water and an organic solvent, such as propylene glycol, ethanol, and the like, or some other sterile injectable medium immediately before use for parenteral administration.

Typical of pharmaceutically acceptable carriers are, for example, manitol, urea, dextrans, lactose, potato and maize starches, magnesium stearate, talc, vegetable oils, polyalkylene glycols, ethyl cellulose, poly(vinylpyrrolidone), calcium carbonate, ethyl oleate, isopropyl myristate, benzyl benzoate, sodium carbonate, gelatin, potassium carbonate, silicic acid. The pharmaceutical preparation may also contain nontoxic auxiliary substances such as emulsifying, preserving, wetting agents, and the like as for example, sorbitan monolaurate, triethanolamine oleate, polyoxyethylene monostearate, glyceryl tripalmitate, dioctyl sodium sulfosuccinate, and the like.

## Claims

**1.** A compound of formula I, or a pharmaceutically acceptable salt thereof

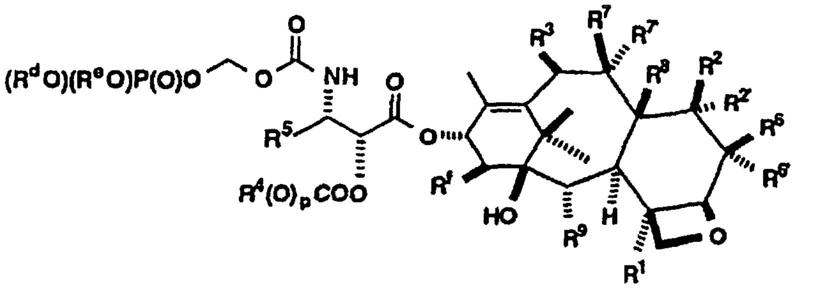

I

wherein $R^1$ is hydroxy, -OC(O)$R^x$ or -OC(O)O$R^x$; $R^2$ is hydrogen, hydroxy, -OC(O)$R^x$ or -OC(O)O$R^x$; $R^{2'}$ is hydrogen, hydroxy or fluoro; $R^{6'}$ is hydrogen or hydroxy; $R^6$ is hydrogen, or $R^2$ and $R^6$ together can form oxirane ring or a bond; $R^3$ is hydrogen, hydroxy, $C_{1-6}$ alkyloxy, -OCON$R^{11}R^{12}$, -OC(O)$R^x$ or -OC(O)O$R^x$; $R^8$ is methyl or hydroxymethyl, or $R^8$ and $R^2$ together can form cyclopropane ring; $R^9$ is hydroxy or -OC(O)$R^x$; with the proviso that when $R^8$ and $R^2$ form cyclopropane ring, $R^{2'}$ is hydrogen; when $R^2$ and $R^6$ form oxirane ring or double bond, $R^{2'}$ and $R^{6'}$ are hydrogen; when $R^2$ is hydroxy, -OC(O)$R^x$ or -OC(O)O$R^x$, $R^{2'}$ is hydrogen; when $R^{2'}$ is fluoro, $R^2$ is hydrogen; one of $R^7$ or $R^{7'}$ is hydrogen and the other is hydroxy, -OC(O)$R^x$ or -OC(O)O$R^x$, or $R^7$ and $R^{7'}$ together can form an oxo group; $R^{11}$ and $R^{12}$ are independently $C_{1-6}$ alkyl, hydrogen, aryl or substituted aryl; $R^4$ and $R^5$ are independently $C_{1-6}$ alkyl, $C_{2-6}$ alkenyl, $C_{2-6}$ alkynyl, or -Z-$R^{10}$; Z is a direct bond, $C_{1-6}$ alkyl or $C_{2-6}$ alkenyl; $R^{10}$ is aryl, substituted aryl, $C_{3-6}$ cycloalkyl or heteroaryl; p is 0 or 1; $R^d$ and $R^e$ are independently hydrogen, $C_{1-6}$ alkyl, aryl, substituted aryl or phosphono protecting group; $R^f$ is hydrogen or hydroxy; $R^x$ is $C_{3-6}$ cycloalkyl, $C_{2-6}$ alkenyl or $C_{1-6}$ alkyl, all can be optionally substituted with one to six same or different halogen atoms; or $R^x$ is a radical of the formula

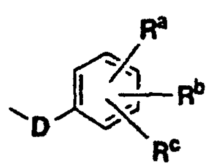

wherein D is a bond or $C_{1-6}$ alkyl; and $R^a$, $R^b$ and $R^c$ are independently hydrogen, nitro, amino, $C_{1-6}$ alkylamino, di-$C_{1-6}$ alkylamino, halogen, $C_{1-6}$ alkyl, hydroxy or $C_{1-6}$ alkoxy.

**2.** A compound of claim 1 wherein $R^1$ is -OC(O)$R^x$; $R^2$ is hydroxy; $R^{2'}$ is hydrogen; $R^3$ is hydroxy or -OC(O)$R^x$; $R^8$ is methyl; $R^6$ and $R^{6'}$ are hydrogen; $R^9$ is -OC(O)$R^x$; $R^7$ and $R^{7'}$ together form an oxo group; $R^4$ and $R^5$ are independently $C_{1-6}$ alkyl, $C_{2-6}$ alkenyl or -Z-$R^{10}$; Z is a direct bond or $C_{1-6}$ alkyl; $R^{10}$ is aryl, substituted aryl, $C_{3-6}$ cycloalkyl or heteroaryl; p is 0; $R^d$ and $R^e$ are hydrogen; $R^f$ is hydrogen; $R^x$ is $C_{3-6}$ cycloalkyl, $C_{2-6}$ alkenyl or $C_{1-6}$ alkyl, all can be optionally substituted with one to six same or different halogen atoms; or $R^x$ is a radical of the formula

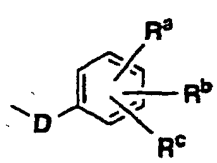

wherein D is a bond or $C_{1-6}$ alkyl; and $R^a$, $R^b$ and $R^c$ are independently hydrogen, nitro, amino, $C_{1-6}$ alkylamino,

di-$C_{1-6}$ alkylamino, halogen, $C_{1-6}$ alkyl or $C_{1-6}$ alkoxy.

3. A compound of claim 2 that is N-debenzoyl-N-[(phosphonooxymethyl)oxy]carbonyl-2-O'-benzoylpaclitaxel.

4. The triethanolamine salt of compound of claim 3.

5. A pharmaceutical formulation which comprises at least one compound of any of claims 1 to 4 and a pharmaceutically acceptable carrier.

6. The use of at least one compound claimed in any of claims 1 to 4 for preparing a pharmaceutical composition for inhibiting tumor growth.

7. The use according to claim 6 wherein the composition is suitable for oral administration.

**Patentansprüche**

1. Verbindung der Formel I oder ein pharmazeutisch verträgliches Salz davon

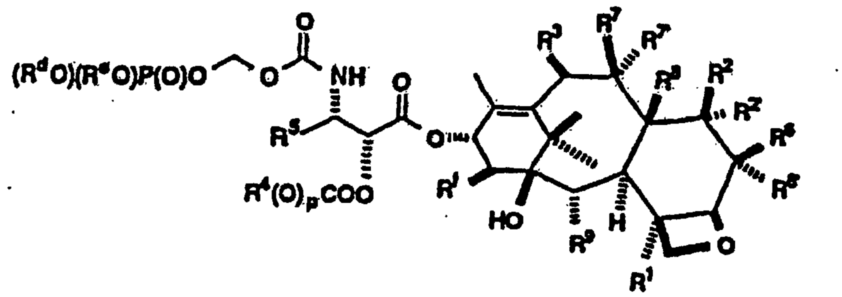

I

worin $R^1$ für Hydroxy, -OC(O)$R^x$ oder -OC(O)O$R^x$ steht; $R^2$ für Wasserstoff, Hydroxy, -OC(O)$R^x$ oder -OC(O)O$R^x$ steht; $R^{2'}$ für Wasserstoff, Hydroxy oder Fluor steht;
$R^{6'}$ für Wasserstoff oder Hydroxy steht; $R^6$ Wasserstoff ist, oder $R^2$ und $R^6$ zusammen einen Oxiranring oder eine Bindung bilden können; $R^3$ für Wasserstoff, Hydroxy, $C_{1-6}$-Alkyloxy, -OCON$R^{11}R^{12}$, -OC(O)$R^x$ oder -OC(O)$R^x$ steht; $R^8$ für Methyl oder Hydroxymethyl steht, oder $R^8$ und $R^2$ zusammen einen Cyclopropanring bilden können; $R^9$ für Hydroxy oder -OC(O)$R^x$ steht; mit der Maßgabe, dass $R^{2'}$ Wasserstoff ist, falls $R^8$ und $R^2$ einen Cyclopropanring bilden; $R^{2'}$ und $R^{6'}$ Wasserstoff sind, falls $R^2$ und $R^6$ einen Oxiranring oder eine Doppelbindung bilden; $R^{2'}$ Wasserstoff ist, falls $R^2$ für Hydroxy, -OC(O)$R^x$ oder -OC(O)O$R^x$ steht; $R^2$ Wasserstoff ist, falls $R^{2'}$ Fluor ist; einer der Reste $R^7$ oder $R^{7'}$ Wasserstoff ist und der andere für Hydroxy, -OC(O)$R^x$ oder -OC(O)O$R^x$ steht, oder $R^7$ und $R^{7'}$ zusammen eine Oxogruppe bilden können; $R^{11}$ und $R^{12}$ unabhängig voneinander für $C_{1-6}$-Alkyl, Wasserstoff, Aryl oder substituiertes Aryl stehen; $R^4$ und $R^5$ unabhängig voneinander für $C_{1-6}$-Alkyl, $C_{2-6}$-Alkenyl, $C_{2-6}$-Alkinyl oder -Z-$R^{10}$ stehen; Z für eine direkte Bindung, $C_{1-6}$-Alkyl oder $C_{2-6}$-Alkenyl steht;
$R^{10}$ für Aryl, substituiertes Aryl, $C_{3-6}$-Cycloalkyl oder Heteroaryl steht; p 0 oder 1 ist; $R^d$ und $R^e$ unabhängig voneinander für Wasserstoff, $C_{1-6}$-Alkyl, Aryl, substituiertes Aryl oder eine Phosphono-Schutzgruppe stehen; $R^f$ für Wasserstoff oder Hydroxy steht; $R^x$ für $C_{3-6}$-Cycloalkyl, $C_{2-6}$-Alkenyl oder $C_{1-6}$-Alkyl steht, wobei alle diese Reste optional mit einem bis 6 gleichen oder verschiedenen Halogenatomen substituiert sein können; oder $R^x$ ein Rest der Formel

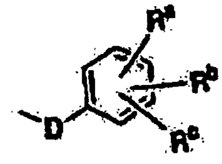

ist, worin D für eine Bindung oder $C_{1-6}$-Alkyl steht; und $R^a$, $R^b$ und $R^c$ unabhängig voneinander für Wasserstoff, Nitro, Amino, $C_{1-6}$-Alkylamino, Di-$C_{1-6}$-alkylamino, Halogen, $C_{1-6}$-Alkyl, Hydroxy oder $C_{1-6}$-Alkoxy steht.

2. Verbindung nach Anspruch 1, worin $R^1$ für -OC(O)$R^x$ steht; $R^2$ Hydroxy ist; $R^{2'}$ Wasserstoff ist; $R^3$ für Hydroxy- oder -OC(O)$R^x$ steht; $R^8$ Methyl ist; $R^6$ und $R^{6'}$ Wasserstoff sind; $R^9$ für -OC(O)$R^x$ steht; $R^7$ und $R^{7'}$ zusammen eine Oxogruppe bilden; $R^4$ und $R^5$ unabhängig voneinander für $C_{1-6}$-Alkyl, $C_{2-6}$-Alkenyl oder -Z-$R^{10}$ stehen; Z für eine direkte Bindung oder $C_{1-6}$-Alkyl steht; $R^{10}$ für Aryl, substituiertes Aryl, $C_{3-6}$-Cycloalkyl oder Heteroaryl steht; p 0 ist; $R^d$ und $R^e$ Wasserstoff sind; $R^f$ Wasserstoff ist; $R^x$ für $C_{3-6}$-Cycloalkyl, $C_{2-6}$-Alkenyl oder $C_{1-6}$-Alkyl steht, wobei alle diese Reste optional mit einem bis 6 gleichen oder verschiedenen Halogenatomen substituiert sein können.; oder $R^x$ ein Rest der Formel

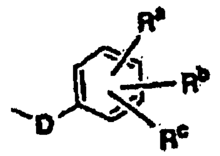

ist, worin D für eine Bindung oder $C_{1-6}$-Alkyl steht; und $R^a$, $R^b$ und $R^c$ unabhängig voneinander für Wasserstoff, Nitro, Amino, $C_{1-6}$-Alkylamino, Di-$C_{1-6}$-alkylamino, Halogen, $C_{1-6}$-Alkyl oder $C_{1-6}$-Alkoxy steht.

3. Verbindung nach Anspruch 2, nämlich N-Debenzoyl-N-[(phosphonooxymethyl)oxy]carbonyl-2-O'-Benzoylpaclitaxel.

4. Triethanolaminsalz der Verbindung nach Anspruch 3.

5. Pharmazeutische Formulierung, die wenigstens eine Verbindung nach einem der Ansprüche 1 bis 4 und einen pharmazeutisch verträglichen Träger umfasst.

6. Verwendung wenigstens einer Verbindung nach einem der Ansprüche 1 bis 4 zur Herstellung einer pharmazeutischen Zusammensetzung zur Inhibierung von Tumorwachstum.

7. Verwendung nach Anspruch 6, wobei die Zusammensetzung für die orale Verabreichung geeignet ist.

**Revendications**

1. Composé de formule I, ou son sel pharmaceutiquement acceptable :

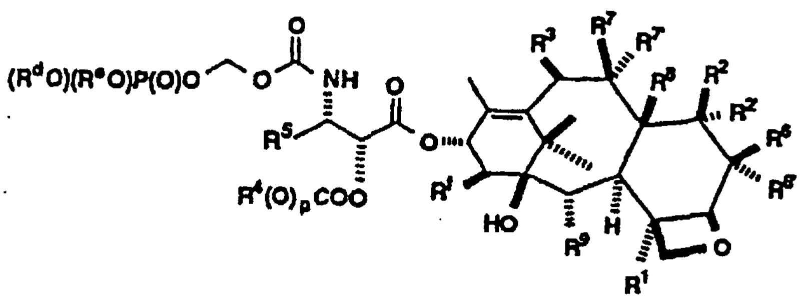

I

où $R^1$ est hydroxy, -OC(O)$R^x$ ou -OC(O)O$R^x$ ; $R^2$ est hydrogène, hydroxy, -OC(O)$R^x$ ou -OC(O)O$R^x$ ; $R^{2'}$ est hydrogène, hydroxy ou fluoro ; $R^{6'}$ est hydrogène ou hydroxy ; $R^6$ est hydrogène, ou bien $R^2$ et $R^6$ ensemble peuvent former un cycle d'oxirane ou une liaison ; $R^3$ est hydrogène, hydroxy, alkyloxy $C_{1-6}$, -OCONR$^{11}$R$^{12}$, -OC(O)$R^x$ ou -OC(O)O$R^x$ ; $R^8$ est méthyle ou hydroxyméthyle, ou bien $R^8$ et $R^2$ peuvent former ensemble un cycle de cyclopropane ; $R^9$ est hydroxy ou -OC(O)$R^x$ ; avec la condition que quand $R^8$ et $R^2$ forment un cycle de cyclopropane, $R^{2'}$ soit hydrogène ; quand $R^2$ et $R^6$ forment un cycle d'oxirane ou une double liaison, $R^{2'}$ et $R^{6'}$ soient hydrogène ; quand $R^2$ est hydroxy, -OC(O)$R^x$ ou -OC(O)O$R^x$, $R^{2'}$ soit hydrogène ; quand $R^{2'}$ est fluoro, $R^2$ soit hydrogène ;l'un de $R^7$ ou $R^{7'}$ est hydrogène et l'autre est hydroxy, -OC(O)$R^x$ ou -OC(O)O$R^x$, ou bien $R^7$ et $R^{7'}$ peuvent former ensemble un groupe oxo ; $R^{11}$ et $R^{12}$ sont indépendamment alkyle $C_{1-6}$, hydrogène, aryle ou aryle substitué ; $R^4$ et $R^5$ sont indépendamment alkyle $C_{1-6}$, alcényle $C_{2-6}$, alkynyle $C_{2-6}$ ou -Z-$R^{10}$ ; Z est une liaison

directe, alkyke $C_{1-6}$ ou alcényle $C_{2-6}$ ; $R^{10}$ est aryle, aryle substitué, cycloalkyle $C_{3-6}$ ou hétéroaryle ; p est 0 ou 1 ; $R^d$ et $R^e$ sont indépendamment hydrogène, alkyle $C_{1-6}$, aryle, aryle substitué ou un groupe phosphono protecteur ; $R^f$ est hydrogène ou hydroxy ; $R^x$ est cycloalkyle $C_{3-6}$, alcényle $C_{2-6}$ ou alkyle $C_{1-6}$, tous peuvent être facultativement substitués par un à six atomes d'halogène identiques ou différents ; ou bien $R^X$ est un radical de la formule

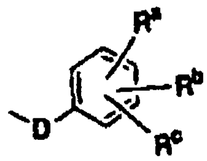

où D est une liaison ou alkyle $C_{1-6}$ ; et $R^a$, $R^b$ et $R^c$ sont indépendamment hydrogène, nitro, amino, alkylamino $C_{1-6}$, di-alkylamino $C_{1-6}$, halogène, alkyle $C_{1-6}$, hydroxy ou alkoxy $C_{1-6}$.

**2.** Composé de la revendication 1 où $R^1$ est -OC(O)$R^x$ ; $R^2$ est hydroxy ; $R^{2'}$ est hydrogène ; $R^3$ est hydroxy ou -OC(O)$R^x$ ; $R^8$ est méthyle; $R^6$ et $R^{6'}$ sont hydrogène ; $R^9$ est -OC(O)$R^x$ ; $R^7$ et $R^{7'}$ forment ensemble un groupe oxo ; $R^4$ et $R^5$ sont indépendamment alkyle $C_{1-6}$, alcényle $C_{2-6}$ ou -Z-$R^{10}$ ; Z est une liaison directe ou alkyle $C_{1-6}$ ; $R^{10}$ est aryle, aryle substitué, cycloalkyle $C_{3-6}$ ou hétéroaryle ; p est 0 ; $R^d$ et $R^e$ sont hydrogène ; $R^f$ est hydrogène ; $R^x$ est cycloalkyle $C_{3-6}$, alcényle $C_{2-6}$ ou alkyle $C_{1-6}$, tous peuvent être facultativement substitués par un à six atomes d'halogène identiques ou différents ; ou bien $R^x$ est un radical de la formule

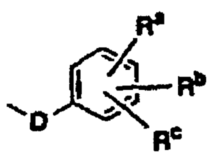

où D est une liaison ou alkyle $C_{1-6}$ ; et $R^a$, $R^b$ et $R^c$ sont indépendamment hydrogène, nitro, amino, alkylamino $C_{1-6}$, di-alkylamino $C_{1-6}$, halogène, alkyle $C_{1-6}$ ou alcoxy $C_{1-6}$.

**3.** Composé de la revendication 2 qui est N-débenzoyl-N-[(phosphonooxyméthyl)oxy]carbonyl-2-O'-benzoylpaclitaxel.

**4.** Sel de triéthanolamine du composé de la revendication 3.

**5.** Formulation pharmaceutique qui comprend au moins un composé de l'une des revendications 1 à 4 et un support pharmaceutiquement acceptable.

**6.** Utilisation d'au moins un composé selon l'une quelconque des revendications 1 à 4 pour la préparation d'une composition pharmaceutique pour l'inhibition de la croissance d'une tumeur.

**7.** Utilisation selon la revendication 6 où la composition est appropriée pour une administration orale.